# EUROPEAN PATENT SPECIFICATION

(11) **EP 3 304 030 B1**
(45) Date of publication and mention of the grant of the patent: **17.03.2021**
(21) Application number: 16804288.5
(22) Date of filing: 01.06.2016
(51) Int. Cl.: G01N 1/30, C12Q 1/02, G01N 33/50, G01N 33/52

(54) **METHODS FOR CELL COUNT AND VIABILITY MEASUREMENTS**
VERFAHREN ZUR ZELLZÄHLUNG UND FÜR LEBENSFÄHIGKEITSMESSUNGEN
PROCÉDÉS DE MESURES DE VIABILITÉ ET DE NOMBRE DE CELLULES

(30) Priority: 01.06.2015 US 201562169537 P
(43) Date of publication of application: 11.04.2018
(73) Proprietor: Nexcelom Bioscience LLC, Lawrence, MA 01843 (US)
(72) Inventor: CHAN, Leo, L., North Andover, MA 01845 (US); QIU, Jean, Andover, MA 01810 (US); PETER, Li, Andover, MA 01810 (US); DERY, Oliver, Lawrence, ME 01843 (US); KESSEL, Sarah, Lawrence, MA 01843 (US); SMITH, Timothy, Lawrence, MA 01843 (US)
(74) Representative: Ter Meer Steinmeister & Partner
(86) International application number: PCT/US2016/035178
(87) International publication number: WO 2016/196567

(56) References cited:
- WO-A1-00/52195
- WO-A1-95/20148
- WO-A1-2012/068446
- WO-A2-02/08454
- WO-A2-2009/126685
- US-A1- 2008 182 290
- US-B1- 6 403 378
- US-B2- 8 570 370
- US-B2- 8 609 363
- MENCHINSKAYA EKATERINA SERGEEVNA.: "MOLEKULYARNYE MEKHANIZMY PROTIVOOPUHOLEVOGO DEYSTVIYA TRITERPENOVYKH GLIKOZIDOV KUKUMARIOZIDA A2-2 I FRONDOZIDA A.", Dissertatsiya na soiskanie uchenoy stspeni kandidata biologicheskikh nauk, 2014, pages 1-128, XP009507712,
- I.G.GERASIMOV et al.: "Otsenka zhiznesposobnosti kletok po ikh morfologicheskim parametram na primere kultiviruemykh fibroblastov.", Tsitologiya, vol. 49, no. 3 , XP009507713,

## Description

### Priority Claims and Related Patent Applications

### Technical Fields of the Invention

The invention generally relates to measurement and analysis of biological samples. More particularly, the invention relates to a novel method for accurate, efficient and high-throughout measurement of cell viability of diverse biological samples.

### Background of the Invention

An important aspect in the fields of medical diagnostics and biomedical research involves detection, identification, quantification, and characterization of various cells and biomolecules of interest through testing of biological samples such as blood, spinal fluid, cell culture and urine. Healthcare providers and biomedical researchers routinely analyze such biological samples for the microscopic presence and concentrations of cells and biomolecules.

Another field experiencing dramatic growth in recent years is biofuel development and production. Currently, the largest biofuel process relies heavily on ethanol production, which utilizes the baker's yeasts, *Saccharomyces cerevisiae,* to perform fermentation on sugar cane, corn meal, polysaccharides, and wastewater. Due to their high ethanol tolerance, final ethanol concentration, glucose conversion rate, and the historical robustness of industrial fermentation, yeasts are the ideal component for bioethanol production. (Antoni, et al. 2007 Appl. Microbiol. Biotech. vol. 77, pp. 23-35; Vertès, et al. 2008 J. Mol. Microbiol. & Biotech. vol. 15, pp. 16-30; Basso, et al. 2008 FEMS Yeast Res. vol. 8, pp. 1155-1163; Nikolic, et al. 2009 J. Chem. Technol. Biotech. vol. 84, pp. 497-503; Gibbons, et al. 2009 In Vitro Cell. & Develop. Biol. - Plant, vol. 45, pp. 218-228; Hu, et al. 2007 Genetics, vol. 175, pp. 1479-1487; Argueso, et al. 2009 Genome Res. vol. 19, pp. 2258-2270; Eksteen, et al. 2003 Biotech. & Bioengin. vol. 84, pp. 639-646.)

There are several methods for concentration and viability measurement, such as dye exclusion and flow cytometry. For example, Trypan Blue, a diazo dye and a vital stain, has been used to selectively color dead tissues or cells blue. Live cells or tissues with intact cell membranes are not colored. Trypan Blue is not absorbed in a viable cell because cells are selective in the compounds that pass through the membrane. However, Trypan Blue traverses the membrane in a dead cell and stain dead cells with a distinctive blue color under a microscope.

Multi-sample cell count and viability analysis method commonly utilizes an image-based platform with automated liquid handling for mixing cell sample with Trypan Blue. The method uses one fixed focal plane to count live cells and Trypan Blue-stained dead cells and generates viability measurement. Measurements based on one focal plane, however, often result in incorrect identification and characterization of cells, leading to inaccurate cell viability and counter measurements.

There is an ongoing need for novel and improved methods that allow accurate, efficient and high-throughput cell viability measurement.

### Summary of the Invention

The invention is based, in part, on the unexpected discovery of an accurate method for efficient and high-throughput cell viability measurement.

The invention features improved an analysis method to automatically capture cell images at distinct focal planes. Importantly, the method of the invention enables and can be readily adapted to high-throughput measurement and analysis. For example, existing analytical systems may be utilized to perform the method of the invention. Significantly, the method of the invention can be employed to perform cell count and viability measurements accurately with high-throughput.

In one aspect, the invention generally relates to a method for measuring cell viability of a biological sample. The method includes: staining a sample to be measured for cell viability with a vital stain; acquiring a first static bright field image of the vital-stained sample at a first focal plane such that substantially all live cells are imaged as exhibiting a first morphological characteristic while substantially all dead cells are imaged as exhibiting a second morphological characteristic; acquiring a second static bright field image of the vital-stained sample at a second focal plane such that substantially all live cells and substantially all dead cells are imaged as exhibiting a third morphological characteristic; and measuring the first static bright field image, and the first and second morphological characteristics therein, and the second static bright field image, and the third morphological characteristic therein, to determine cell viability of the biological sample. The method of the present invention is defined in claim 1.

In another aspect, the invention generally relates to a method for simultaneously measuring cell viabilities for multiple biological samples. The method includes: providing a plurality of samples to be measured for cell viability in a plurality of individually addressable wells; staining each of the plurality of samples with one or more vital stains; simultaneously acquiring a first set of static bright field images of the vital-stained samples at a first focal plane such that substantially all live cells are imaged as exhibiting a first morphological characteristic while substantially all dead cells are imaged as exhibiting a second morphological characteristic; simultaneously acquiring a second set of static bright field images of the vital-stained sample at a second focal plane such that substantially all live cells and substantially all dead cells are imaged as exhibiting a third morphological characteristic; and measuring the first sets of static bright field images, and the first and second morphological characteristics therein, and the second sets of static bright field images, and the third morphological characteristics therein, to determine cell viability for each of the plurality of samples.

In yet another aspect, the invention generally relates to a method for measuring cell viability of a biological sample. The method includes: staining a sample to be measured for cell viability with a vital stain; acquiring a first static bright field image of the vital-stained sample at a first focal plane such that substantially all live cells are imaged as bright centers while substantially all dead cells are imaged as dark spots; acquiring a second static bright field image of the vital-stained sample at a second focal plane such that substantially all live cells and substantially all dead cells are imaged as dark spots; and measuring the first static bright field image and the second static bright field image to determine cell viability of the biological sample.

In yet another aspect, the invention generally relates to a method for simultaneously measuring cell viabilities for multiple biological samples. The method includes: providing a plurality of samples to be measured for cell viability in a plurality of individually addressable wells; staining each of the plurality of samples with one or more vital stains; simultaneously acquiring a first set of static bright field images of the vital-stained samples at a first focal plane such that substantially all live cells are imaged as bright centers while substantially all dead cells are imaged as dark spots; simultaneously acquiring a second set of static bright field images of the vital-stained sample at a second focal plane such that substantially all live cells and substantially all dead cells are imaged as dark spots; and measuring the first sets of static bright field images and the second sets of static bright field images to determine cell viability for each of the plurality of samples.

Further described is a method for simultaneously measuring cell concentration and cell viability of a biological sample.

### Brief Description of the Drawings

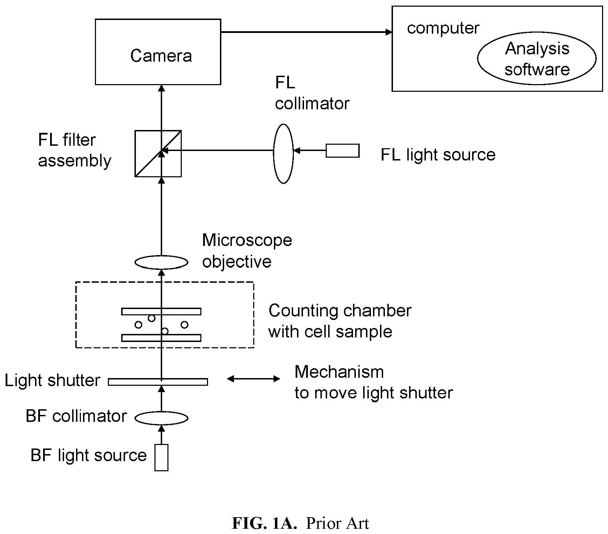

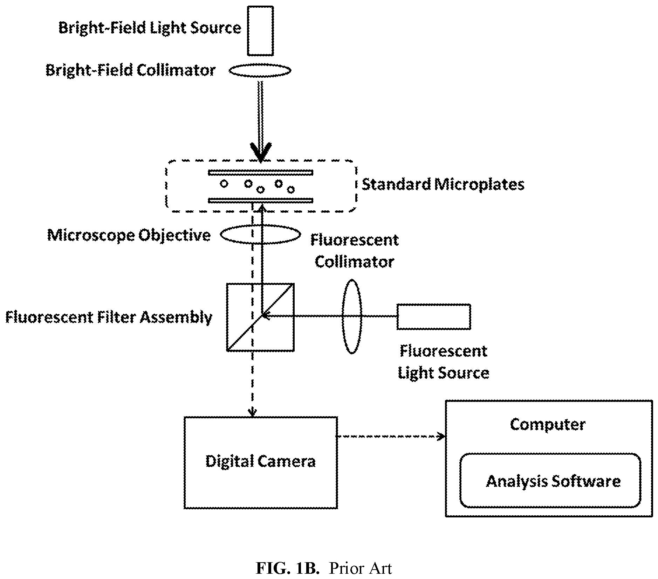

**FIG. 1. (A).** Exemplary imaging system (Vision Cellometer®, Nexcelom Bioscience LLC, Lawrence, MA). (Prior Art). [
**FIG. 1. (B)** Exemplary imaging system (Celigo®, Nexcelom Bioscience LLC, Lawrence, MA). (Prior Art).
**FIG. 2.** (A) Exemplary image taken from Focal Plane 1, where live cells were shown as bright centers and dead cells were shown as dark spots (stained with Trypan Blue).

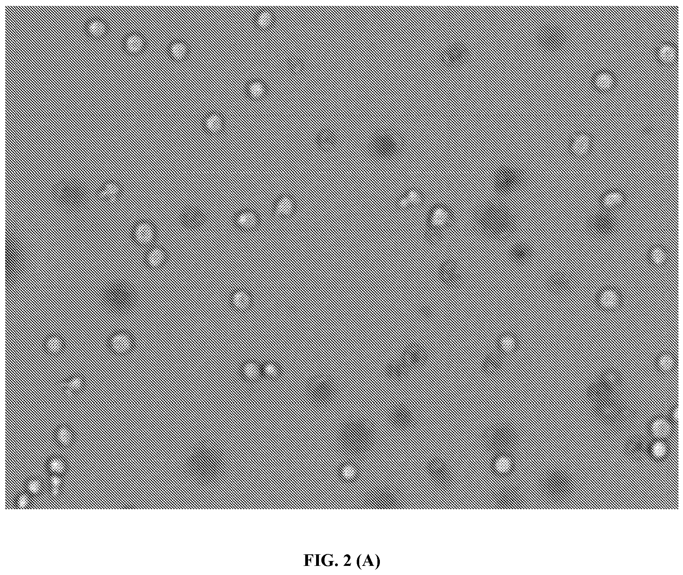

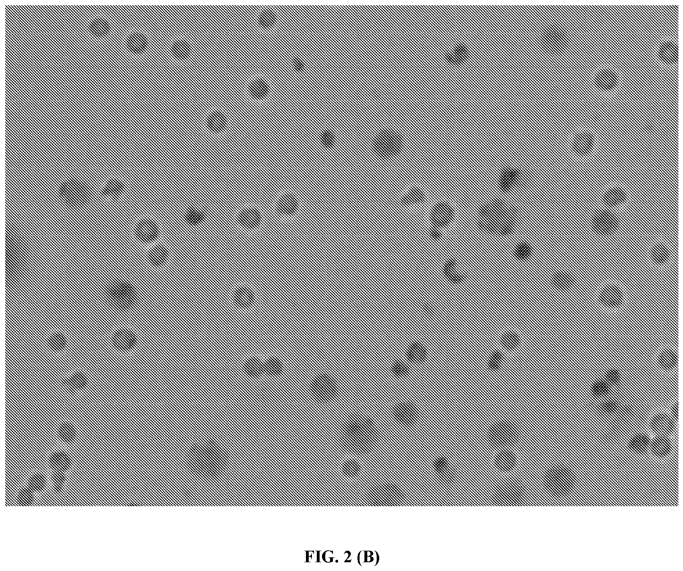

**FIG. 2.** (B) Exemplary image taken from Focal Plane 2, where all cells (live and dead) are focused to show in a dark color.

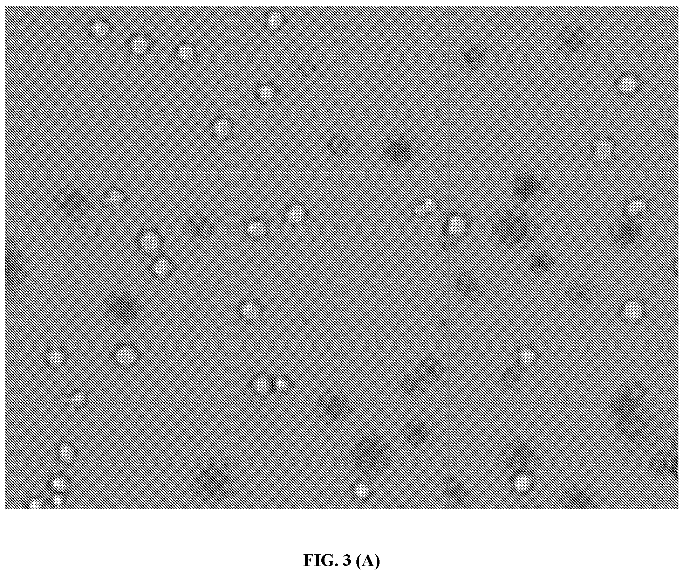

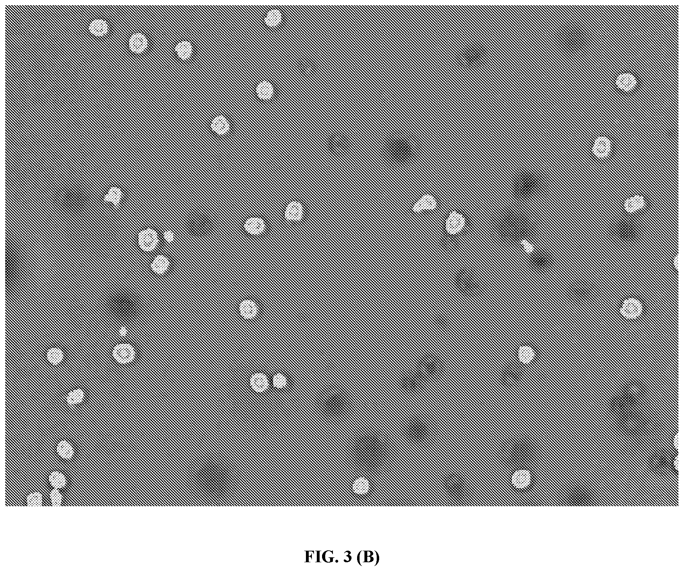

**FIG. 3.** (A) Exemplary captured bright-field image of Trypan Blue stained Jurkat cells at Focal Plane 1.
**FIG. 3.** (B) Exemplary counted bright-field image of live Jurkat cells as bright center.

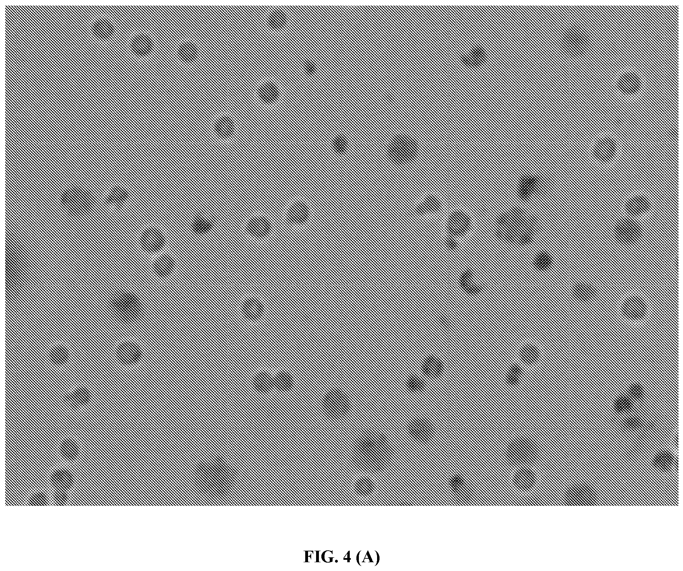

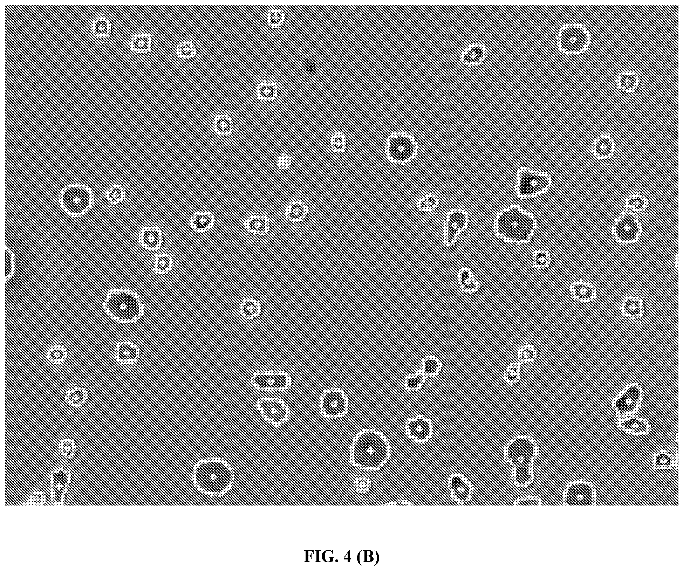

**FIG. 4.** (A) Exemplary captured bright-field image of Trypan blue stained Jurkat cells at Focal Plane 2.
**FIG. 4.** (B) Exemplary counted bright-field image of total (live and dead) Jurkat cells as dark color.

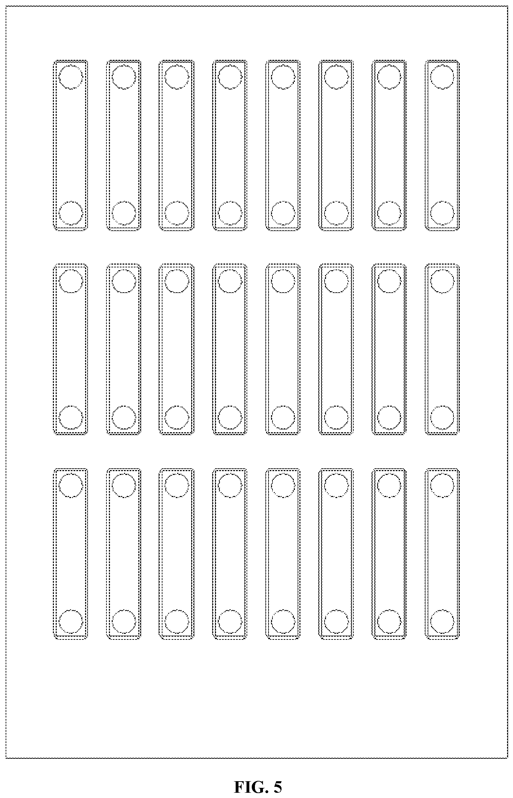

**FIG. 5****.** Schematic illustration of an exemplary multi-sample chamber.

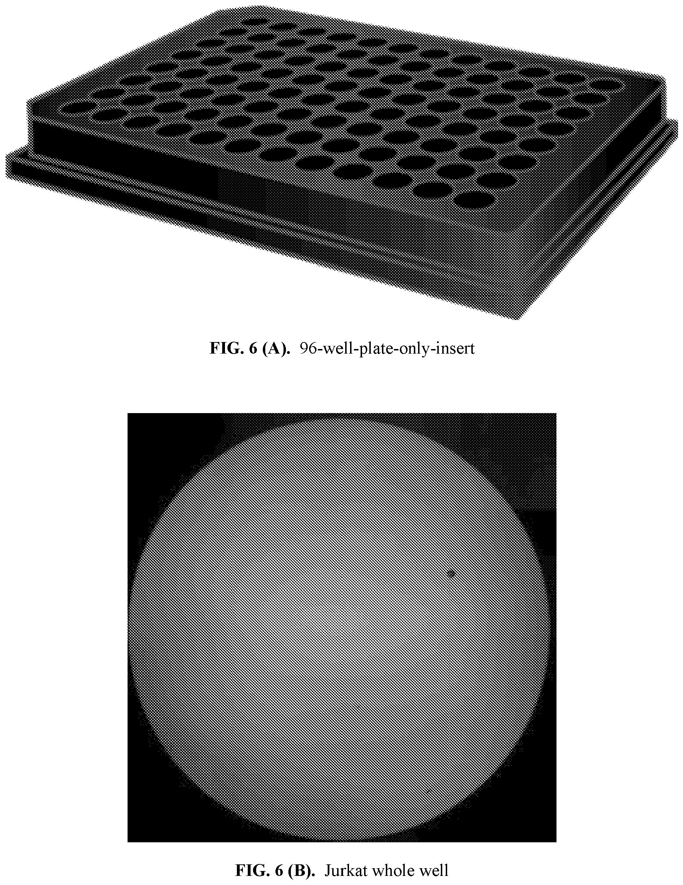

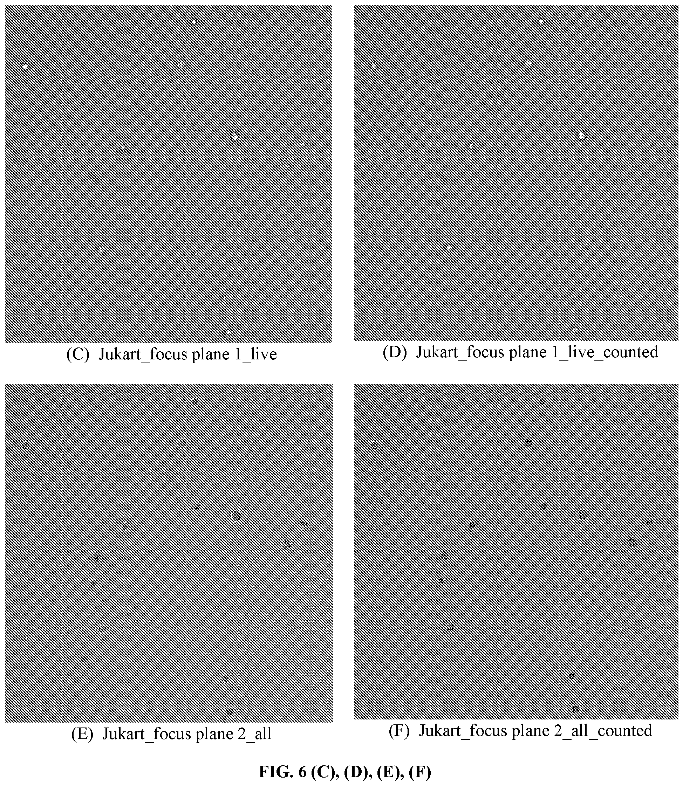

**FIG. 6.** (A)-(F) Schematic illustration, exemplary images and count cells in a 96-well plate.

### Detailed Description of the Invention

The invention provides an accurate method for efficient and high-throughput cell viability measurement.

The invention features improved an analysis method, for example using the Cellometer® and Celigo® imaging cytometer (Nexcelom Bioscience LLC, Lawrence, MA) to automatically capture cell images at distinct focal planes. For example, Trypan Blue-stained cell images are automatically captured at a first focal plane (Plane 1) with definitive live cells with bright centers, while the dead cells are dark spots. Cell images are again automatically captured at a second focal plane (Plane 2), where all of the cells are dark spots allowing the total cell count to be measured. Therefore, the numbers of live and total cells can be used to calculate the viability of the biological sample.

Importantly, the method of the invention enables and can be readily adapted to high-throughput measurement and analysis. For example, existing analytical systems may be utilized to perform the method of the invention (*e.g.,* Celigo® imaging cytometer). A variety of vessels and sizes can be employed, ranging from standard microplates (6, 12, 24, 48, 96, 384, 1536-wells), cell culture flasks (T25, T75) and glass chambers, for example. When the method of the invention is used with single and multi-sample counting chambers with controlled (fixed and known) height, accurate measurement of cell concentration can be achieved because the volume of sample under imaging can be accurately assessed. (See, US Pat. Nos. 8,883,491; 9,342,734; 9,329,130; 8,928,876; 9,186,843; and 9,075,790).

According to an exemplary embodiment of the invention, cell count, concentration and viability can be measured by analyzing bright-field images of Trypan Blue-stained cell samples.

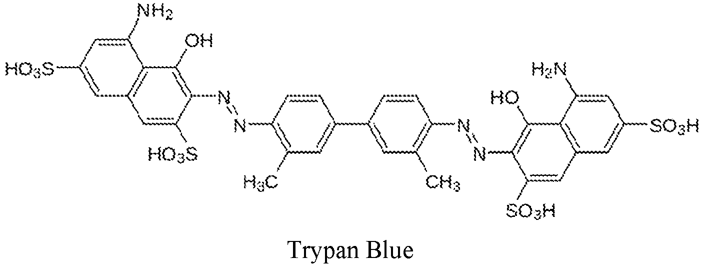

In one aspect, the invention generally relates to a method for measuring cell viability of a biological sample. The method includes: staining a sample to be measured for cell viability with a vital stain; acquiring a first static bright field image of the vital-stained sample at a first focal plane such that substantially all live cells are imaged as exhibiting a first morphological characteristic while substantially all dead cells are imaged as exhibiting a second morphological characteristic; acquiring a second static bright field image of the vital-stained sample at a second focal plane such that substantially all live cells and substantially all dead cells are imaged as exhibiting a third morphological characteristic; and measuring the first static bright field image, and the first and second morphological characteristics therein, and the second static bright field image, and the third morphological characteristic therein, to determine cell viability of the biological sample.

In certain embodiments, the first, second and third morphological characteristic is independently selected from bright center, dark spot, a select size, and a select shape. In certain embodiments, the dark spot is selected from a diffused dark spot and a tight dark spot. In certain embodiments, the select shape is selected from a diffused circular shape, a shriveled shape, and an elongated shape.

In certain preferred embodiments, the first morphological characteristic is a bright center spot in the bright field image, the second morphological characteristic is a dark spot in the bright field image, and the third morphological characteristic is a dark spot in the bright field image.

Any suitable vital stain may be utilized, for example, Trypan Blue, Methylene Blue (methylthioninium chloride) or Crystal Violet (hexamethyl-p-rosaniline chloride).

In certain preferred embodiments, the vital stain is Methylene Blue. In certain preferred embodiments, the vital stain is Crystal Violet.

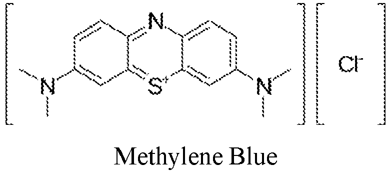

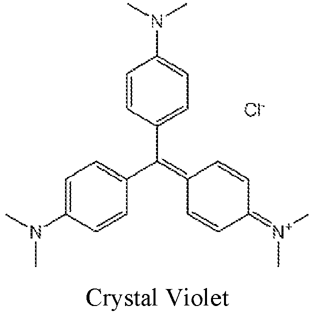

In certain preferred embodiments, the vital stain is Trypan Blue. In certain embodiments, the Trypan Blue-stained cells has a concentration from about 1 cell/mL to about 5 million cells/mL (*e.g.,* from about 1 cell/mL to about 1 million cells/mL, from about 1 cell/mL to about 500,000 cells/mL, from about 1 cell/mL to about 100,000 cells/mL, from about 1 cell/mL to about 5 million cells/mL, from about 1 cell/mL to about 10,000 cells/mL, from about 1,000 cells/mL to about 5 million cells/mL, from about 10,000 cells/mL to about 5 million cells/mL, from about 100,000 cells/mL to about 5 million cells/mL, from about 1 million cells/mL to about 5 million cells/mL). In certain embodiments, the Trypan Blue-stained cells have a concentration from about 1 cell/mL to about 10,000 cells/mL.

In certain embodiments, the first static bright field image obtained from the first focal plane captures greater than about 95% of all live cells as exhibiting the first morphological characteristic and greater than about 95% of dead cells as exhibiting the second morphological characteristic. In certain preferred embodiments, the first static bright field image obtained from the first focal plane captures greater than about 99% of all live cells as exhibiting the first morphological characteristic and greater than about 99% of dead cells as exhibiting the second morphological characteristic. In certain preferred embodiments, the first static bright field image obtained from the first focal plane captures greater than about 99.9% of all live cells as exhibiting the first morphological characteristic and greater than about 99.9% of dead cells as exhibiting the second morphological characteristic.

In certain embodiments, the second static bright field image obtained from the first focal plane captures greater than about 95% of all live and dead cells as exhibiting the third morphological characteristic. In certain preferred embodiments, the second static bright field image obtained from the first focal plane captures greater than about 99% of all live and dead cells as exhibiting the third morphological characteristic. In certain preferred embodiments, the second static bright field image obtained from the first focal plane captures greater than about 99.9% of all live and dead cells as exhibiting the third morphological characteristic.

Any suitable biological cells may be measured or analyzed utilizing the invention disclosed herein. In certain embodiments, the sample to be tested for cell viability comprises cells selected from human cancer cell type (NCI 60) and mammalian cells. In certain embodiments, the sample to be tested for cell viability is a sample selected from cell culture, primary mammalian cells and human cells. In certain embodiments, the sample to be tested for cell viability is a sample of human cells.

In another aspect, the invention generally relates to a method for simultaneously measuring cell viabilities for multiple biological samples. The method includes: providing a plurality of samples to be measured for cell viability in a plurality of individually addressable wells; staining each of the plurality of samples with one or more vital stains; simultaneously acquiring a first set of static bright field images of the vital-stained samples at a first focal plane such that substantially all live cells are imaged as exhibiting a first morphological characteristic while substantially all dead cells are imaged as exhibiting a second morphological characteristic; simultaneously acquiring a second set of static bright field images of the vital-stained sample at a second focal plane such that substantially all live cells and substantially all dead cells are imaged as exhibiting a third morphological characteristic; and measuring the first sets of static bright field images, and the first and second morphological characteristics therein, and the second sets of static bright field images, and the third morphological characteristics therein, to determine cell viability for each of the plurality of samples.

In certain embodiments, each of the first, second and third morphological characteristic is independently selected from bright center, dark spot, a select size, and a select shape.

In certain embodiments, the dark spot is selected from a diffused dark spot and a tight dark spot. In certain embodiments, the select shape is selected from a diffused circular shape, a shriveled shape, and an elongated shape. In certain preferred embodiments, the first morphological characteristic is a bright center spot in the bright field image, the second morphological characteristic is a dark spot in the bright field image, and the third morphological characteristic is a dark spot in the bright field image.

In certain embodiments, the vital stain is selected from Trypan Blue, Methylene Blue and Crystal Violet. In certain preferred embodiments, the vital stain is Methylene Blue. In certain preferred embodiments, the vital stain is Crystal Violet.

In certain preferred embodiments, the vital stain is Trypan Blue. In certain embodiments, the Trypan Blue-stained cells has a concentration from about 1 cell/mL to about 5 million cells/mL (e.g., from about 1 cell/mL to about 1 million cells/mL, from about 1 cell/mL to about 500,000 cells/mL, from about 1 cell/mL to about 100,000 cells/mL, from about 1 cell/mL to about 5 million cells/mL, from about 1 cell/mL to about 10,000 cells/mL, from about 1,000 cells/mL to about 5 million cells/mL, from about 10,000 cells/mL to about 5 million cells/mL, from about 100,000 cells/mL to about 5 million cells/mL, from about 1 million cells/mL to about 5 million cells/mL). In certain embodiments, the Trypan Blue-stained cells have a concentration from about 1 cell/mL to about 10,000 cells/mL.

In certain preferred embodiments, each of the first static bright field image obtained from the first focal plane captures greater than about 95% of all live cells as exhibiting the first morphological characteristic and greater than about 95% of dead cells as exhibiting the second morphological characteristic. In certain preferred embodiments, each of the first static bright field image obtained from the first focal plane captures greater than about 99% of all live cells as exhibiting the first morphological characteristic and greater than about 99% of dead cells as exhibiting the second morphological characteristic. In certain preferred embodiments, each of the first static bright field image obtained from the first focal plane captures greater than about 99.9% of all live cells as exhibiting the first morphological characteristic and greater than about 99.9% of dead cells as exhibiting the second morphological characteristic.

In certain embodiments, each of the second static bright field image obtained from the first focal plane captures greater than about 95% of all live and dead cells as exhibiting the third morphological characteristic. In certain preferred embodiments, each of the second static bright field image obtained from the first focal plane captures greater than about 99% of all live and dead cells as exhibiting the third morphological characteristic. In certain preferred embodiments, each of the second static bright field image obtained from the first focal plane captures greater than about 99.9% of all live and dead cells as exhibiting the third morphological characteristic.

Any suitable biological cells may be measured or analyzed utilizing the invention disclosed herein. In certain embodiments, the samples to be tested for cell viability comprises cells selected from human cancer cell type (NCI 60) and mammalian cells. In certain embodiments, the samples to be tested for cell viability are selected from cell culture, primary mammalian cells and human cells. In certain embodiments, the samples to be tested for cell viability are samples of human cells.

In yet another aspect, the invention generally relates to a method for measuring cell viability of a biological sample. The method includes: staining a sample to be measured for cell viability with a vital stain; acquiring a first static bright field image of the vital-stained sample at a first focal plane such that substantially all live cells are imaged as bright centers while substantially all dead cells are imaged as dark spots; acquiring a second static bright field image of the vital-stained sample at a second focal plane such that substantially all live cells and substantially all dead cells are imaged as dark spots; and measuring the first static bright field image and the second static bright field image to determine cell viability of the biological sample.

In certain embodiments, the first static bright field image obtained from the first focal plane captures greater than about 95% of all live cells as bright centers and greater than about 99% of dead cells as dark spots. In certain preferred embodiments, the first static bright field image obtained from the first focal plane captures greater than about 99% of all live cells as bright centers and greater than about 99% of dead cells as dark spots. In certain preferred embodiments, the first static bright field image obtained from the first focal plane captures greater than about 99.9% of all live cells as bright centers and greater than about 99.9% of dead cells as dark spots.

In certain embodiments, the second static bright field image obtained from the first focal plane captures greater than about 95% of all live and dead cells as dark spots. In certain preferred embodiments, the second static bright field image obtained from the first focal plane captures greater than about 99% of all live and dead cells as dark spots. In certain preferred embodiments, the second static bright field image obtained from the first focal plane captures greater than about 99.9% of all live and dead cells as dark spots.

In yet another aspect, the invention generally relates to a method for simultaneously measuring cell viabilities for multiple biological samples. The method includes: providing a plurality of samples to be measured for cell viability in a plurality of individually addressable wells; staining each of the plurality of samples with one or more vital stains; simultaneously acquiring a first set of static bright field images of the vital-stained samples at a first focal plane such that substantially all live cells are imaged as bright centers while substantially all dead cells are imaged as dark spots; simultaneously acquiring a second set of static bright field images of the vital-stained sample at a second focal plane such that substantially all live cells and substantially all dead cells are imaged as dark spots; and measuring the first sets of static bright field images and the second sets of static bright field images to determine cell viability for each of the plurality of samples.

In certain embodiments, each of the first static bright field image obtained from the first focal plane captures greater than about 95% of all live cells as exhibiting the first morphological characteristic and greater than about 99% of dead cells as exhibiting the second morphological characteristic. In certain preferred embodiments, each of the first static bright field image obtained from the first focal plane captures greater than about 99% of all live cells as exhibiting the first morphological characteristic and greater than about 99% of dead cells as exhibiting the second morphological characteristic. In certain preferred embodiments, each of the first static bright field image obtained from the first focal plane captures greater than about 99.9% of all live cells as exhibiting the first morphological characteristic and greater than about 99.9% of dead cells as exhibiting the second morphological characteristic.

In certain embodiments, each of the second static bright field image obtained from the first focal plane captures greater than about 95% of all live and dead cells as exhibiting the third morphological characteristic. In certain preferred embodiments, each of the second static bright field image obtained from the first focal plane captures greater than about 99% of all live and dead cells as exhibiting the third morphological characteristic. In certain preferred embodiments, each of the second static bright field image obtained from the first focal plane captures greater than about 99.9% of all live and dead cells as exhibiting the third morphological characteristic.

In certain embodiments, the method disclosed herein further includes measuring a cell count of live cells.

In certain embodiments, the method disclosed herein further includes measuring a concentration of live and/or dead cells.

In certain embodiments, the method disclosed herein further includes the biological sample is imaged in a cell chamber having a fixed and known height allowing measurement of the volume of the biological sample being imaged.

### Examples

### Materials and instrumentation

Fresh Jurkat cells were collected from a T75 cell culture flask at approximately 2 x 10⁶ cells/mL. The Trypan Blue viability stain was prepared to a working concentration ranging from 0.2% to 0.04%. The Celigo® imaging cytometer was set up to take two bright-field channels, where one channel was used to take images at one focal plane, and the other channel was used to take images at the second focal plane.

In order to develop an improved image-based Trypan Blue viability analysis, Jurkat cells were stained at different concentration of Trypan blue ranging from 0.1% - 0.02% final. The cells were pipetted into standard 96-well microplate and Nexcelom multi-sample chamber **(****FIG. 5****)** for image acquisition. **FIG. 5** is a multi-sample chamber slide that can measure 24 samples made using optically clear polymer.

The images were captured at a first focal plane with high contrast for live cells and a second focal plane with all cells exhibiting a dark color. The images were analyzed for total live cells at focal Plane 1, and total cells for Focal Plane 2. The viability results were then directly calculated from the counted images.

### Initial Trypan Blue concentration test

Fresh Jurkat cells were stained with different concentrations of Trypan Blue, and 200 µL of the stained cells were pipetted into the 96-well plate. The plate was then scanned using Celigo® to examine the captured bright-field images. The test was to verify the optimal Trypan Blue staining concentration for Celigo®.

### Viability analysis method using 2-focal planes

After identifying the optimal Trypan Blue staining concentration, the same concentration was used to stain different concentration of Jurkat cells. The stained cell samples were pipetted into the 96-well plate and scanned using Celigo® at two different focal planes. The test was to determine the feasibility of measuring viability using the 2-Focal Plane detection method.

### Testing viability in different vessels

Cells can be contained in flasks, microplates, and enclosed chambers made with plastic or glass. In this experiment, Trypan Blue stained Jurkat cells were tested in 96-well microplate to measure total cell count and viability. In addition, they were tested in an enclosed Nexcelom multi-sample chamber plate. The multi-sample chamber plate is constructed with plastic at a fixed height, thus the final counted cell number can be used to generate an accurate concentration and viability of the tested sample.

### Initial Trypan Blue concentration test

The initial Trypan blue concentration test showed dark images for concentrations at between 0.06 - 0.1% final concentrations. The optimal image for viability analysis was approximately 0.02% staining concentration **(****FIG. 2A** and **FIG. 2B****).**

### Viability analysis method using 2-focal planes

The live and total cells were counted rapidly using the Celigo® analysis software. In this experiment, the live cells with bright center were counted in Focal Plane 1, which was shown in **FIG. 3A** and **FIG. 3B****.** In addition, the dark dead cells were not counted.

The total cell count was achieved by focusing on a second plane, where all the cells were dark. **FIG. 4A** and **FIG. 4B** show the dark characteristics of each cell and how they were counted.

### Testing viability in different vessels

The viability can be measured from the example images shown above. The additional viability measurement was shown with Jurkat cells in the Nexcelom multi-chamber plate. The results showed that viability and concentration can be successfully measured from a multi-chamber plate. The results are shown in **TABLE 1.** The table shows the cell counting and viability results from the multi-sample chamber slide. It shows that the results are highly consistent, and repeatable for using trypan blue to measure viability. This allows the user to quickly measure concentration and viability using the two focal plane method.

**Table 1.**

| % Viability | 1 | 2 | 3 | | % Viability | 1 | 2 | 3 |
|---|---|---|---|---|---|---|---|---|
| A | 92% | 84% | 50% | | A | 10% | 39% | |
| B | 90% | 80% | 45% | | B | 12% | 39% | |
| C | 90% | 84% | 45% | | C | 11% | 43% | |
| D | 93% | 85% | 51% | | D | 14% | 40% | |
| E | 93% | 90% | 59% | | E | 12% | 55% | |
| F | 94% | 90% | 73% | | F | 11% | 76% | |
| G | 92% | 90% | 82% | | G | 12% | 72% | |
| H | 93% | 79% | 70% | | H | 11% | 40% | |
| | | | | | | | | |
| Average | 92% | 85% | 60% | | Average | 12% | 51% | |
| STDEV | 2% | 4% | 14% | | STDEV | 1% | 15% | |
| %CV | 2% | 5% | 24% | | %CV | 11% | 31% | |
| | | | | | | | | |
| Live | 1 | 2 | 3 | | Live | 1 | 2 | |
| A | 3.81E+06 | 4.50E+06 | 2.20E+06 | | A | 4.25E+05 | 1.89E+06 | |
| B | 3.77E+06 | 4.00E+06 | 1.95E+06 | | B | 4.74E+05 | 2.09E+06 | |
| C | 3.77E+06 | 4.37E+06 | 1.95E+06 | | C | 4.13E+05 | 2.26E+06 | |
| D | 3.89E+06 | 4.22E+06 | 2.27E+06 | | D | 5.40E+05 | 1.84E+06 | |
| E | 3.91E+06 | 4.51E+06 | 2.91E+06 | | E | 4.57E+05 : | 2.82E+06 | |
| F | 3.96E+06 | 4.45E+06 | 3.53E+06 | | F | 4.47E+05 | 3.91E+06 | |
| G | 3.98E+06 | 4.29E+06 | 3.92E+06 | | G | 4.43E+05 | 3.69E+06 | |
| H | 3.92E+06 | 3. 68E+06 : | 3.50E+06 | | H | 4.33E+05 : | 2.11E+06 | |
| | | | | | | | | |
| | | | | | | | | |
| Dead | 1 | 2 | 3 | | Dead | 1 | | |
| A | 3.02E+05 | 8. 54E+05 : | 2.21E+06 | | A | 3.67E+06 | 2.93E+06 | |
| B | 4.44E+05 | 9. 68E+05 | 2.36E+06 | | B | 3.33E+06 | 3.28E+06 | |
| C | 4.44E+05 | 8.20E+05 | 2.36E+06 | | C | 3.32E+06 | 2.95E+06 | |
| D | 3.09E+05 | 7. 72E+05 | 2.14E+06 | | D | 3.19E+06 | 2.75E+06 | |
| E | 2.76E+05 | 4.90E+05 | 2.04E+06 | | E | 3.21E+06 | 2.34E+06 | |
| F | 2.54E+05 | 4.70E+05 | 1.30E+06 | | F | 3.57E+06 | 1.21E+06 | |
| G | 3.31E+05 | 4. 98E+05 | 8.47E+05 | | G | 3.30E+06 | 1.43E+06 | |
| H | 2.86E+05 | 9.70E+05 | 1.48E+06 | | H | 3.46E+06 | 3.14E+06 | |
| | | | | | | | | |
| | | | | | | | | |
| Total | 1 | 2 | 3 | | Total | 1 | 2 | 3 |
| A | 4.12E+06 | 5.36E+06 | 4.41E+06 | | A | 4.10E+06 | 4.82E+06 | |
| B | 4.21E+06 | 4.97E+06 | 4.30E+06 | | B | 3.S0E+06 | 5.37E+06 | |
| C | 4.21E+06 | 5. 19E+06 : | 4.30E+06 | | C | 3.73E+06 | 5.21E+06 | |
| D | 4.20E+06 | 4.99E+06 | 4.41E+06 | | D | 3.73E+06 | 4.59E+06 | |
| E | 4.9E+06 | 5.00E+06 | 4.95E+06 | | E | 3.67E+06 | 5.16E+06 | |
| F | 4.21E+06 | 4.92E+06 | 4.83E+06 | | F | 4.02E+06 | 5.12E+06 | |
| G | 4.31E+06 | 4.79E+06 | 4.76E+06 | | G | 3.74E+06 | 5.12E+06 | |
| H | 4.21E+06 | 4. 65E+06 | 4.98E+06 | | H | 3.89E+06 | 5.25E+06 | |

### High-throughput Measurement

The current methods can only perform 12 samples in ∼30 min, while the Celigo® imaging cytometer can perform 96 samples in less than 5 min, which drastically increase throughput. (Bug, et al. 2011 "Anthracyclines induce the Acculumation of Mutant p53 Through E2F1-Dependant and Independent Mechanisms" Oncogene, 30 (22):3612-24.)

**FIG. 6** shows schematic illustration, exemplary images and count cells in a 96-well plate. (A) Illustration of a 96-well plate. (B) Whole well image of Jukat cells stained with Trypin Blue. (C) Zoomed in image of Trypan Blue stained Jukat cells in focus plane 1 for counting bright centered live cells. (D) Zoomed in image of Trypan Blue stained Jukat cells in focus plane 1 with counted, bright centered live cells, indicated by the green circles. (E) Zoomed in image of Trypan Blue stained Jukat cells in focus plane 2 for counting all of the cells. (F) Zoomed in image of Trypan Blue stained Jukat cells in focus plane 2 with counted all types of cells, indicated by the red circles.

Applicant's disclosure is described herein in preferred embodiments with reference to the Figures, in which like numbers represent the same or similar elements. Reference throughout this specification to "one embodiment," "an embodiment," or similar language means that a particular feature, structure, or characteristic described in connection with the embodiment is included in at least one embodiment of the present invention. Thus, appearances of the phrases "in one embodiment," "in an embodiment," and similar language throughout this specification may, but do not necessarily, all refer to the same embodiment.

The described features, structures, or characteristics of Applicant's disclosure may be combined in any suitable manner in one or more embodiments. In the following description, numerous specific details are recited to provide a thorough understanding of embodiments of the invention. One skilled in the relevant art will recognize, however, that Applicant's composition and/or method may be practiced without one or more of the specific details, or with other methods, components, materials, and so forth. In other instances, well-known structures, materials, or operations are not shown or described in detail to avoid obscuring aspects of the disclosure.

In this specification and the appended claims, the singular forms "a," "an," and "the" include plural reference, unless the context clearly dictates otherwise.

Unless defined otherwise, all technical and scientific terms used herein have the same meaning as commonly understood by one of ordinary skill in the art. Although any methods and materials similar or equivalent to those described herein can also be used in the practice or testing of the present disclosure, the preferred methods and materials are now described. Methods recited herein may be carried out in any order that is logically possible, in addition to a particular order disclosed.

References and citations to other documents, such as patents, patent applications, patent publications, journals, books, papers, web contents, have been made in this disclosure.

### Equivalents

The representative examples are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples and the references to the scientific and patent literature included herein. The examples contain important additional information, exemplification and guidance that can be adapted to the practice of this invention in its various embodiments and equivalents thereof.

## Claims

1. A method for measuring cell viability of a biological sample, comprising:
staining a sample to be measured for cell viability with a vital stain;
acquiring a first static bright field image of the vital-stained sample at a first focal plane such that substantially all live cells are imaged as exhibiting a first morphological characteristic while substantially all dead cells are imaged as exhibiting a second morphological characteristic;
acquiring a second static bright field image of the vital-stained sample at a second focal plane such that substantially all live cells and substantially all dead cells are imaged as exhibiting a third morphological characteristic; and
measuring the first static bright field image, and the first and second morphological characteristics therein, and the second static bright field image, and the third morphological characteristic therein, to determine cell viability of the biological sample, wherein each of the first, second and third morphological characteristics is independently selected from a bright center, a dark spot, a select size, and a select shape.

2. The method of Claim 1, wherein the dark spot is selected from a diffused dark spot and a tight dark spot.

3. The method of Claim 1, wherein the select shape is selected from a diffused circular shape, a shriveled shape, and an elongated shape.

4. The method of Claim 1, wherein the first morphological characteristic is a bright center spot in the bright field image, the second morphological characteristic is a dark spot in the bright field image, and the third morphological characteristic is a dark spot in the bright field image.

5. The method of any of Claims 1-4, wherein the vital stain is selected from Trypan Blue, Methylene Blue and Crystal Violet.

6. The method of Claim 5, wherein the vital stain is Trypan Blue.

7. The method of Claim 6, wherein Trypan Blue-stained cells has a concentration from 1 cell/mL to 5 million cells/mL.

8. The method of any of Claims 1-7, wherein the first static bright field image obtained from the first focal plane captures greater than about 99% of all live cells as exhibiting the first morphological characteristic and greater than about 99% of dead cells as exhibiting the second morphological characteristic.

9. The method of any of Claims 1-8, wherein the second static bright field image obtained from the first focal plane captures greater than about 99% of all live and dead cells as exhibiting the third morphological characteristic.

10. The method of any of Claims 1-9, wherein the sample to be tested for cell viability comprises cells selected from human cancer cell type (NCI 60) and mammalian cells.

11. The method of any of Claims 1-10, wherein the sample to be tested for cell viability is a sample selected from cell culture, primary mammalian cells and human cells.

12. A method for simultaneously measuring cell viabilities for multiple biological samples, comprising:
providing a plurality of samples to be measured for cell viability in a plurality of individually addressable wells;
staining each of the plurality of samples with one or more vital stains;
simultaneously acquiring a first set of static bright field images of the vital-stained samples at a first focal plane such that substantially all live cells are imaged as exhibiting a first morphological characteristic while substantially all dead cells are imaged as exhibiting a second morphological characteristic;
simultaneously acquiring a second set of static bright field images of the vital-stained sample at a second focal plane such that substantially all live cells and substantially all dead cells are imaged as exhibiting a third morphological characteristic; and
measuring the first sets of static bright field images, and the first and second morphological characteristics therein, and the second sets of static bright field images, and the third morphological characteristics therein, to determine cell viability for each of the plurality of samples, wherein each of the first, second and third morphological characteristics is independently selected from a bright center, a dark spot, a select size, and a select shape.

13. A method for measuring cell viability of a biological sample as defined in claim 1, comprising:
staining a sample to be measured for cell viability with a vital stain;
acquiring a first static bright field image of the vital-stained sample at a first focal plane such that substantially all live cells are imaged as bright centers while substantially all dead cells are imaged as dark spots;
acquiring a second static bright field image of the vital-stained sample at a second focal plane such that substantially all live cells and substantially all dead cells are imaged as dark spots; and
measuring the first static bright field image and the second static bright field image to determine cell viability of the biological sample.

14. A method for simultaneously measuring cell viabilities for multiple biological samples as defined in claim 12, comprising:
providing a plurality of samples to be measured for cell viability in a plurality of individually addressable wells;
staining each of the plurality of samples with one or more vital stains;
simultaneously acquiring a first set of static bright field images of the vital-stained samples at a first focal plane such that substantially all live cells are imaged as bright centers while substantially all dead cells are imaged as dark spots;
simultaneously acquiring a second set of static bright field images of the vital-stained sample at a second focal plane such that substantially all live cells and substantially all dead cells are imaged as dark spots; and
measuring the first sets of static bright field images and the second sets of static bright field images to determine cell viability for each of the plurality of samples.

## Patentansprüche

1. Verfahren zur Messung der Zelllebensfähigkeit einer biologischen Probe, umfassend:
Färben einer Probe, deren Zelllebensfähigkeit gemessen werden soll, mit einem Vital-Farbstoff;
Erfassen eines ersten statischen Hellfeldbildes der vitalgefärbten Probe in einer ersten Brennebene, so dass im Wesentlichen alle lebenden Zellen so abgebildet werden, dass sie ein erstes morphologisches Merkmal aufweisen, während im Wesentlichen alle toten Zellen so abgebildet werden, dass sie ein zweites morphologisches Merkmal aufweisen;
Erfassen eines zweiten statischen Hellfeldbildes der vitalgefärbten Probe in einer zweiten Fokusebene, so dass im Wesentlichen alle lebenden Zellen und im Wesentlichen alle toten Zellen so abgebildet werden, dass sie ein drittes morphologisches Merkmal aufweisen; und
Messen des ersten statischen Hellfeldbildes und der ersten und zweiten morphologischen Charakteristiken darin und des zweiten statischen Hellfeldbildes und der dritten morphologischen Charakteristik darin, um die Zelllebensfähigkeit der biologischen Probe zu bestimmen, wobei jede der ersten, zweiten und dritten morphologischen Charakteristiken unabhängig voneinander aus einem hellen Zentrum, einem dunklen Fleck, einer ausgewählten Größe und einer ausgewählten Form ausgewählt ist.

2. Verfahren nach Anspruch 1, wobei der dunkle Fleck aus einem diffusen dunklen Fleck und einem engen dunklen Fleck ausgewählt ist.

3. Verfahren nach Anspruch 1, wobei die ausgewählte Form aus einer diffusen kreisförmigen Form, einer geschrumpften Form und einer länglichen Form ausgewählt ist.

4. Verfahren nach Anspruch 1, wobei das erste morphologische Merkmal ein heller zentraler Fleck in dem Hellfeldbild ist, das zweite morphologische Merkmal ein dunkler Fleck in dem Hellfeldbild ist und das dritte morphologische Merkmal ein dunkler Fleck in dem Hellfeldbild ist.

5. Verfahren nach einem der Ansprüche 1-4, wobei die Vitalfärbung ausgewählt ist aus Trypanblau, Methylenblau und Kristallviolett.

6. Verfahren nach Anspruch 5, wobei die Vitalfärbung Trypanblau ist.

7. Verfahren nach Anspruch 6, wobei die mit Trypanblau gefärbten Zellen eine Konzentration von 1 Zelle/ml bis 5 Millionen Zellen/ml aufweisen.

8. Verfahren nach einem der Ansprüche 1 bis 7, wobei das erste statische Hellfeldbild, das von der ersten Fokusebene erhalten wird, mehr als etwa 99 % aller lebenden Zellen, die das erste morphologische Merkmal aufweisen, und mehr als etwa 99 % der toten Zellen, die das zweite morphologische Merkmal aufweisen, erfasst.

9. Verfahren nach einem der Ansprüche 1 bis 8, wobei das zweite statische Hellfeldbild, das von der ersten Fokusebene erhalten wird, mehr als etwa 99 % aller lebenden und toten Zellen erfasst, die das dritte morphologische Merkmal aufweisen.

10. Verfahren nach einem der Ansprüche 1-9, wobei die auf Zelllebensfähigkeit zu testende Probe Zellen umfasst, die aus menschlichem Krebszelltyp (NCI 60) und Säugetierzellen ausgewählt sind.

11. Verfahren nach einem der Ansprüche 1-10, wobei die auf Zelllebensfähigkeit zu testende Probe eine Probe ist, die aus Zellkultur, primären Säugetierzellen und menschlichen Zellen ausgewählt ist.

12. Verfahren zur gleichzeitigen Messung der Zelllebensfähigkeit für mehrere biologische Proben, umfassend
Bereitstellen einer Vielzahl von Proben, die auf Zelllebensfähigkeit gemessen werden sollen, in einer Vielzahl von einzeln adressierbaren Vertiefungen;
Färben jeder der Vielzahl von Proben mit einer oder mehreren Vitalitätsfärbungen; gleichzeitiges Erfassen eines ersten Satzes von statischen Hellfeldbildern der vitalgefärbten Proben in einer ersten Fokusebene, so dass im Wesentlichen alle lebenden Zellen so abgebildet werden, dass sie ein erstes morphologisches Merkmal aufweisen, während im Wesentlichen alle toten Zellen so abgebildet werden, dass sie ein zweites morphologisches Merkmal aufweisen;
gleichzeitiges Erfassen eines zweiten Satzes von statischen Hellfeldbildern der vitalgefärbten Probe in einer zweiten Fokusebene, so dass im Wesentlichen alle lebenden Zellen und im Wesentlichen alle toten Zellen so abgebildet werden, dass sie ein drittes morphologisches Merkmal aufweisen; und
Messen des ersten Satzes von statischen Hellfeldbildern und der ersten und zweiten morphologischen Charakteristiken darin und des zweiten Satzes von statischen Hellfeldbildern und der dritten morphologischen Charakteristiken darin, um die Zelllebensfähigkeit für jede der Vielzahl von Proben zu bestimmen, wobei jede der ersten, zweiten und dritten morphologischen Charakteristiken unabhängig aus einem hellen Zentrum, einem dunklen Fleck, einer ausgewählten Größe und einer ausgewählten Form ausgewählt wird.

13. Verfahren zur Messung der Zelllebensfähigkeit einer biologischen Probe, wie in Anspruch 1 definiert, umfassend:
Färben einer Probe, deren Zelllebensfähigkeit gemessen werden soll, mit einer Vitalfärbung;
Erfassen eines ersten statischen Hellfeldbildes der vitalgefärbten Probe in einer ersten Brennebene, so dass im Wesentlichen alle lebenden Zellen als helle Zentren abgebildet werden, während im Wesentlichen alle toten Zellen als dunkle Flecken abgebildet werden;
Erfassen eines zweiten statischen Hellfeldbildes der vitalgefärbten Probe in einer zweiten Brennebene, so dass im Wesentlichen alle lebenden Zellen und im Wesentlichen alle toten Zellen als dunkle Flecken abgebildet werden; und
Messen des ersten statischen Hellfeldbildes und des zweiten statischen Hellfeldbildes, um die Zelllebensfähigkeit der biologischen Probe zu bestimmen.

14. Verfahren zur gleichzeitigen Messung der Zelllebensfähigkeit für mehrere biologische Proben gemäß Anspruch 12, umfassend
Bereitstellen einer Vielzahl von Proben, die auf Zelllebensfähigkeit gemessen werden sollen, in einer Vielzahl von einzeln adressierbaren Vertiefungen;
Färben jeder der Vielzahl von Proben mit einer oder mehreren Vitalfärbungen gleichzeitiges Erfassen eines ersten Satzes von statischen Hellfeldbildern der vitalgefärbten Proben in einer ersten Fokusebene, so dass im Wesentlichen alle lebenden Zellen als helle Zentren abgebildet werden, während im Wesentlichen alle toten Zellen als dunkle Flecken abgebildet werden;
gleichzeitiges Erfassen eines zweiten Satzes von statischen Hellfeldbildern der vitalgefärbten Probe in einer zweiten Fokusebene, so dass im Wesentlichen alle lebenden Zellen und im Wesentlichen alle toten Zellen als dunkle Flecken abgebildet werden; und
Messen des ersten Satzes von statischen Hellfeldbildern und des zweiten Satzes von statischen Hellfeldbildern, um die Zelllebensfähigkeit für jede der Vielzahl von Proben zu bestimmen.

## Revendications

1. Procédé pour mesurer la viabilité cellulaire d'un échantillon biologique, comprenant:
la coloration d'un échantillon à mesurer quant à la viabilité cellulaire avec un colorant vital;
l'acquisition d'une première image statique en champ clair de l'échantillon coloré vital dans un premier plan focal, de telle sorte que pratiquement toutes les cellules vivantes sont représentées comme présentant une première caractéristique morphologique, tandis que pratiquement toutes les cellules mortes sont représentées comme présentant une deuxième caractéristique morphologique ;
l'acquisition d'une deuxième image statique en champ clair de l'échantillon coloré de façon vitale dans un deuxième plan focal, de telle sorte que pratiquement toutes les cellules vivantes et pratiquement toutes les cellules mortes soient représentées comme présentant une troisième caractéristique morphologique ; et
mesurer la première image statique en champ clair, et les première et deuxième caractéristiques morphologiques qu'elle contient, et la deuxième image statique en champ clair, et la troisième caractéristique morphologique qu'elle contient, pour déterminer la viabilité cellulaire de l'échantillon biologique, dans lequel chacune des première, deuxième et troisième caractéristiques morphologiques est indépendamment sélectionnée parmi un centre clair, un point sombre, une taille sélectionnée et une forme sélectionnée.

2. Procédé selon la revendication 1, dans lequel le point sombre est sélectionnée parmi un point sombre diffuse et un point sombre serré.

3. Procédé selon la revendication 1, dans lequel la forme sélectionnée est choisie parmi une forme circulaire diffuse, une forme ratatinée et une forme allongée.

4. Procédé selon la revendication 1, dans lequel la première caractéristique morphologique est un point central clair dans l'image en champ clair, la deuxième caractéristique morphologique est un point sombre dans l'image en champ clair, et la troisième caractéristique morphologique est un point sombre dans l'image en champ clair.

5. Procédé selon l'une quelconque des revendications 1 à 4, dans lequel le colorant vital est choisie parmi le bleu de trypan, le bleu de méthylène et le violet de cristal.

6. Procédé selon la revendication 5, dans lequel le colorant vital est le bleu trypan.

7. Procédé selon la revendication 6, dans lequel les cellules colorées au bleu de trypan ont une concentration de 1 cellule/mL à 5 millions de cellules/mL.

8. Procédé selon l'une quelconque des revendications 1 à 7, dans lequel la première image statique en champ clair obtenue à partir du premier plan focal capture plus d'environ 99 % de toutes les cellules vivantes comme présentant la première caractéristique morphologique et plus d'environ 99 % des cellules mortes comme présentant la deuxième caractéristique morphologique.

9. Procédé selon l'une des revendications 1-8, dans lequel la deuxième image statique en champ clair obtenue à partir du premier plan focal capture plus d'environ 99% de toutes les cellules vivantes et mortes comme présentant la troisième caractéristique morphologique.

10. Procédé selon l'une des revendications 1 à 9, dans lequel l'échantillon à tester quant à la viabilité cellulaire comprend des cellules choisies parmi le type de cellules cancéreuses humaines (NCI 60) et des cellules de mammifères.

11. Procédé selon l'une quelconque des revendications 1 à 10, dans lequel l'échantillon dont la viabilité cellulaire doit être testée est un échantillon choisi parmi une culture cellulaire, des cellules primaires de mammifères et des cellules humaines.

12. Procédé pour mesurer simultanément la viabilité des cellules pour de multiples échantillons biologiques, comprenant :
fournir une pluralité d'échantillons à mesurer quant à la viabilité cellulaire dans une pluralité de puits adressables individuellement ;
la coloration de chacun des échantillons avec une ou plusieurs taches vitales ;
l'acquisition simultanée d'un premier ensemble d'images statiques en champ clair des échantillons vitaux colorés dans un premier plan focal, de sorte que pratiquement toutes les cellules vivantes sont imagées comme présentant une première caractéristique morphologique, tandis que pratiquement toutes les cellules mortes sont imagées comme présentant une deuxième caractéristique morphologique ;
l'acquisition simultanée d'une deuxième série d'images statiques en champ clair de l'échantillon coloré de cellules vitales dans un deuxième plan focal, de telle sorte que la quasi-totalité des cellules vivantes et la quasi-totalité des cellules mortes soient considérées comme présentant une troisième caractéristique morphologique ; et
mesurer les premiers ensembles d'images statiques en champ clair, et les premières et deuxièmes caractéristiques morphologiques qu'ils contiennent, et les deuxièmes ensembles d'images statiques en champ clair, et les troisièmes caractéristiques morphologiques qu'ils contiennent, afin de déterminer la viabilité des cellules pour chacun de la pluralité d'échantillons, chacune des premières, deuxièmes et troisièmes caractéristiques morphologiques étant indépendamment sélectionnée parmi un centre clair, un point sombre, une taille sélectionnée et une forme sélectionnée.

13. Procédé pour mesurer la viabilité cellulaire d'un échantillon biologique tel que défini dans la revendication 1, comprenant :
la coloration d'un échantillon à mesurer quant à la viabilité cellulaire avec un colorant vital;
l'acquisition d'une première image statique en champ clair de l'échantillon coloré vital à un premier plan focal de telle sorte que pratiquement toutes les cellules vivantes sont imagées comme des centres clairs tandis que pratiquement toutes les cellules mortes sont imagées comme des taches sombres ;
l'acquisition d'une deuxième image statique en champ clair de l'échantillon coloré de façon vitale dans un deuxième plan focal, de telle sorte que pratiquement toutes les cellules vivantes et pratiquement toutes les cellules mortes soient représentées sous forme de points sombres ; et
en mesurant la première image statique en champ clair et la deuxième image statique en champ clair pour déterminer la viabilité cellulaire de l'échantillon biologique.

14. Procédé pour mesurer simultanément la viabilité cellulaire de plusieurs échantillons biologiques, tel que défini dans la revendication 12, comprenant :
fournir une pluralité d'échantillons à mesurer quant à la viabilité cellulaire dans une pluralité de puits adressables individuellement ;
la coloration de chacun des échantillons de la pluralité d'échantillons avec une ou plusieurs taches vitales ;
l'acquisition simultanée d'un premier ensemble d'images statiques en champ clair des échantillons vitaux colorés dans un premier plan focal de telle sorte que pratiquement toutes les cellules vivantes soient imagées comme des centres clairs tandis que pratiquement toutes les cellules mortes sont imagées comme des points sombres ;
l'acquisition simultanée d'une deuxième série d'images statiques en champ clair de l'échantillon coloré de façon vitale dans un deuxième plan focal, de telle sorte que pratiquement toutes les cellules vivantes et pratiquement toutes les cellules mortes soient représentées sous forme de points sombres ; et
en mesurant les premiers ensembles d'images statiques en champ clair et les seconds ensembles d'images statiques en champ clair pour déterminer la viabilité des cellules pour chacun de la pluralité d'échantillons.
